# EUROPÄISCHE PATENTSCHRIFT

(11) **EP 2 701 573 B1**
(45) Veröffentlichungstag und Bekanntmachung des Hinweises auf die Patenterteilung: **22.08.2018**
(21) Anmeldenummer: 12717292.2
(22) Anmeldetag: 26.04.2012
(51) Int. Cl.: A61B 1/00, A61B 1/05, G02B 23/24

(54) **ENDOSKOP**
ENDOSCOPE
ENDOSCOPE

(30) Priorität: 26.04.2011 DE 102011017536
(43) Veröffentlichungstag der Anmeldung: 05.03.2014
(73) Patentinhaber: Otto-von-Guericke Universität Magdeburg Medizinische Fakultät, 39120 Magdeburg (DE)
(72) Erfinder: ARENS, Christoph, 39120 Magdeburg (DE); BOESE, Axel, 39106 Magdeburg (DE)
(74) Vertreter: Kailuweit & Uhlemann Patentanwälte Partnerschaft mbB
(86) Internationale Anmeldenummer: PCT/EP2012/057667
(87) Internationale Veröffentlichungsnummer: WO 2012/146664

(56) Entgegenhaltungen:
- EP-A1- 1 683 472
- EP-A1- 1 759 629
- WO-A1-2005/120330
- WO-A1-2010/097315
- WO-A2-00/57770
- DE-A1-102005 015 522
- DE-U1- 20 113 031
- US-B1- 6 371 909

## Beschreibung

Die Erfindung betrifft Endoskope.

Die heute im Einsatz befindlichen Endoskope haben alle eine eingeschränkte Beweglichkeit und können keine Rundumsicht anbieten. In den meisten Fällen gibt es starre Winkel-Endoskope. Die flexiblen Endoskope können nur bedingt frei gesteuert werden und sind gerade im Bereich ihres beweglichen Schaftes sehr anfällig für Korrosion. Derzeit ist im Bereich der starren Endoskopie noch die Stablinsen-Optik das Maß aller Dinge.

Im Bereich der flexiblen Endoskope setzen sich zunehmend die Chip-on-the-tip-Endoskope durch. Trotzdem ist die Qualität der Stablinsen-Optiken mit aufgesetzter Drei-Chip-Kamera besser als die alleinigen Chip-on-the-tip-Endoskope.

Es besteht eine eingeschränkte Beweglichkeit und Flexibilität der Endoskope.

Durch die Druckschrift EP 1 759 629 A1 ist ein Endoskop mit einem langgestreckten starren Schaft und einem optischen Abbildungssystem bekannt. Das optische Abbildungssystem ist dabei bezüglich des Schaftes um zumindest eine zweite Schwenkachse verschwenkbar ist, die von einer ersten Schwenkachse beabstandet ist und etwa quer zur Längsachse des Schaftes verläuft. Neben der Drehachse sind zwei unabhängig voneinander zu bedienende Schwenkachsen vorhanden.

Die Druckschrift DE 201 13 031 U1 umfasst ein Videoendoskop mit einer drehbaren Videokamera. Die drehbare Videokamera ist mittels Schleifkontakten elektrisch mit einem ortsfesten Teil des Videoendoskops verbunden.

Die Druckschrift WO 2010 / 097 315 A1 offenbart eine räumliche Steuerung eines bildgebenden endoskopischen Instrumentes, wobei das distale Ende des Instrumentes abhängig von der Anzeige der jeweiligen Position und vorgesehenen Aktion von einer motorisch angetriebenen Einrichtung gehaltert und bewegt wird. Dabei werden die Augenbewegungen der Bedienungsperson oder die Blickachse bei der Beobachtung der Anzeige nach der Eye-Tracking-Methode erfasst und zur Steuerung der Bildaufnahme und der Bildnachführung sowie der Anzeige verwendet.

Der im Patentanspruch 1 angegebenen Erfindung liegt die Aufgabe zugrunde, Objekte mit einem Endoskop einfach zu betrachten.

Diese Aufgabe wird mit den im Patentanspruch 1 aufgeführten Merkmalen gelöst.

Die Endoskope zeichnen sich insbesondere dadurch aus, dass Objekte mit den Endoskopen einfach und ohne Bewegung der Endoskope an sich betrachtbar sind.

Dazu befindet sich ein drehbar gelagerter Halter mit einer Digitalkamera in einer drehbar gelagerten Gabel. Dabei ist die Gabel radial drehbar so angeordnet, dass die Drehachsen der Halterung und des Halters senkrecht zueinander angeordnet sind.

Mittels einer Schubkurbel wenigstens bereichsweise in einem rohrförmigen Trägerelement der Gabel ist der Halter mit der Digitalkamera schwenkbar. Das Endoskop weist weiterhin einen ersten rotatorischen und ringförmigen Schrittmotor, wobei sowohl der Stator als auch der Rotor des Schrittmotors in Form eines Elektromotors ringförmig ausgebildet sind, für das rohrförmige Trägerelement und damit der Gabel und einen zweiten an die Schubkurbel gekoppelten translatorischen Schrittmotor für den Halter auf, wobei
- der translatorische Schrittmotor mit der Schubkurbel und das rohrförmige Trägerelement an den an den Rotor des ersten rotatorischen und ringförmigen Schrittmotor gekoppelt sind,
- sich das Anschlusskabel für die Digitalkamera wenigstens bereichsweise in der Schubkurbel und damit im Inneren des Endoskops befindet und
- die Schrittmotore mit einer Steuereinrichtung verbunden sind, so dass die Positionen der Gabel und des Halters sowie daraus die Position der Bildfläche der Digitalkamera bestimmbar sind.

Darüber hinaus besitzt das Endoskop eine Haube aus einem für sichtbares Licht transparentem Material für die drehbar gelagerte Gabel einschließlich dem Halter mit der Digitalkamera, wobei die Abmessungen des Endoskopschafts nicht verändert sind.

Damit ist die Digitalkamera in zwei Freiheitsgraden zu bewegen, wobei die Drehachsen senkrecht zueinander angeordnet sind. Über die Digitalkamera ist bis auf den Träger damit eine Rundumsicht möglich.

Die Digitalkamera zeichnet sich vorteilhafterweise dadurch aus, dass das auf dem Bildsensor fallende Abbild gleichzeitig digital gewandelt wird. Die digitalen Daten lassen sich vielseitig verwenden. Diese können einfach gespeichert, verarbeitet und/oder sichtbar dargestellt werden. Das erfolgt vorteilhafterweise mittels eines bekannten Datenverarbeitungssystems beispielsweise in Form eines Mikrorechners.

Günstigerweise weist das Endoskop den ersten rotatorischen und ringförmigen Antrieb für das rohrförmige Trägerelement auf, wobei sowohl der Stator als auch der Rotor des Antriebs in Form eines Elektromotors ringförmig ausgebildet sind.

Die Daten können so vorteilhafterweise von der Digitalkamera über das Anschlusskabel im Träger und einem Griffstück des Endoskops am Ende des Endoskops abgenommen werden. Beispielsweise kann das Anschlusskabel weitergeführt sein oder kann an einem Verbinder am Griffstück enden, wobei das Anschlusskabel mit dem Verbinder verbunden ist.

Durch die Schrittmotore sind die Positionen der Gabel und des Halters sowie daraus die Position der Bildfläche der Digitalkamera mittels einer mit den Schrittmotoren verbundenen Steuereinrichtung bestimmbar. In Verbindung mit einem Maßstab als Bestandteil des Endoskops sind damit auch Längen und daraus folgernd Größen bestimmbar.

Das Anschlusskabel kann als flexible Leiterplatte mit Leiterbahnen ausgeführt sein. Damit ist eine Beweglichkeit bei den Bewegungen der Digitalkamera und der Gabel gegeben. Das kann auch durch eine wenigstens bereichsweise spiral- und/oder schraubenförmige Anordnung des Anschlusskabels gewährleistet werden.

Das Anschlusskabel kann dabei entweder wenigstens eine Datenleitung oder wenigstens eine Datenleitung sowie eine Energieleitung umfassen.

Damit ist ein sicherer Betrieb der Digitalkamera gewährleistet. Die Daten können verlustfrei, ohne Längenbegrenzung und einfach an ein Datenverarbeitungssystem weitergeleitet werden.

Das Endoskop zeichnet sich weiterhin durch ein deutlich erweitertes Gesichtsfeld aus, das den Operateur in die Lage versetzt, in hoher Auflösung eine optimale minimal-invasive Chirurgie durchführen zu können. Abmessungen des Endoskopschafts werden nicht verändert.
Die Haube sichert, dass das Endoskop abgeschlossen und damit leicht desinfizierbar ist.

Vorteilhafte Ausgestaltungen der Erfindung sind in den Patentansprüchen 2 bis 10 angegeben.

An der Gabel und/oder dem Halter befindet sich nach der Weiterbildung des Patentanspruchs 2 wenigstens ein Leuchtmittel für sichtbares Licht. Vorteilhafterweise wird durch das Leuchtmittel der Aufnahmebereich der Digitalkamera ausgeleuchtet. Dadurch ist ein kompaktes System zur Untersuchung vorhanden. Neben Leuchtmitteln für weißes Licht können auch Leuchtmittel für Licht anderer Farben in Form elektromagnetischer Strahlung einer bestimmten Wellenlänge eingesetzt werden. Natürlich sind auch Kombinationen verschiedener Leuchtmittel oder Leuchtmittel für mehrere Wellenlängen elektromagnetischer Strahlung zur Anwendung kommen. Die Sichtbarmachung bestimmter Objekte wird erhöht.

Das Anschlusskabel ist nach der Weiterbildung des Patentanspruchs 3 günstigerweise eine flexible Leiterplatte, die einen flexiblen Träger und Leiterbahnen aufweist.

Nach der Weiterbildung des Patentanspruchs 4 sind die Schrittmotore mit wenigstens einer fußbetätigbaren Schalteinrichtung verbunden. Der Nutzer des Endoskops steuert die Position der Digitalkamera über die fußbetätigbare Schalteinheit, so dass seine Hände für weitere Tätigkeiten frei sind.

Nach der Weiterbildung des Patentanspruchs 5 ist eine Steuereinrichtung mit den Schrittmotoren und einer Vorrichtung zur Blickerfassung verbunden, so dass die Schrittmotore durch die Bewegung des Auges gesteuert wird und die durch die Digitalkamera abgebildete Fläche mittels der Augenposition bestimmt ist. Die Vorrichtung zur Blickerfassung ist auch als Eyetracker bekannt. Wenigstens eine Augenkamera trägt der Nutzer mittels einer speziellen Vorrichtung. Das kann auch eine Brille sein. Damit wird die Hand nicht zur Steuerung der Digitalkamera benötigt.

Eine Steuereinrichtung ist nach der Weiterbildung des Patentanspruchs 6 mit den Schrittmotoren und einer Einrichtung zur Gestenerkennung verbunden, so dass die Schrittmotore durch die Bewegung eines Körperteiles gesteuert werden.

Die Gestenerkennung basiert auf Sensoren, die von einer Person getragen werden, oder mindestens einer Kamera, die die Bewegungen einer Person aufnimmt.

Der Halter weist nach der Weiterbildung des Patentanspruchs 7 gleichzeitig elektrisch leitende Elemente wenigstens zum Betrieb der Digitalkamera auf. Das sind wenigstens leitende Bestandteile zur Stromversorgung der Digitalkamera.

Die Haube ist nach der Weiterbildung des Patentanspruchs 8 eine Kugelkappe. Weiterhin sind die Kugelkappe und ein Träger dicht miteinander verbunden. Die dichte Verbindung erlaubt eine einfache Säuberung und Desinfizierung des Endoskops, ohne das Bauteile davon beeinflusst werden.

Wenigstens ein Bereich des Trägers ist nach der Weiterbildung des Patentanspruchs 9 ein starrer oder flexibler Bereich.
Ein Ausführungsbeispiel der Erfindung ist in den Zeichnungen jeweils prinzipiell dargestellt und wird im Folgenden näher beschrieben.

Es zeigen:
Fig. 1 ein Endoskop,
Fig. 2 eine drehbar gelagerte Gabel mit einem drehbar gelagerten Halter mit einer Digitalkamera,
Fig. 3 Antriebe und ein Zugmittelgetriebe für die Gabel und den Halter,
Fig. 4 ein Griffstück mit Antrieben,
Fig. 5 eine drehbar gelagerte Gabel mit einem drehbar gelagerten Halter mit einer Digitalkamera und
Fig. 6 einen drehbar gelagerten Halter als Schubgelenk.

Ein Endoskop besteht im Wesentlichen aus einem Griffstück 1, einem rohrförmigen Träger 2 und einer Haube 3.

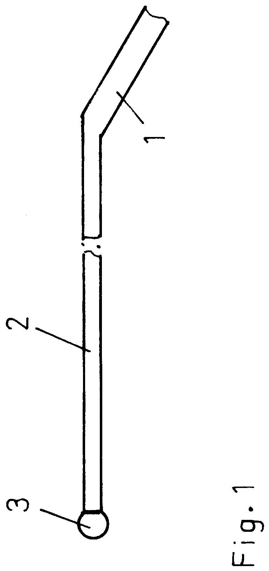

Die Fig. 1 zeigt ein Endoskop in einer prinzipiellen Darstellung.

Weitere Bestandteile des Endoskops sind eine Digitalkamera 4, ein Halter 5, eine Gabel 6, ein Übertragungsmechanismus und wenigstens ein Antriebsmechanismus.

Der Halter 5 mit der Digitalkamera 4 ist drehbar zwischen den Schenkeln der Gabel 6 angeordnet. Die Gabel 6 ist radial drehbar mit dem rohrförmigen Träger 2 verbunden, so dass die Drehachsen der Gabel 6 und des Halters 5 senkrecht zueinander angeordnet sind. Das kann günstigerweise gleichzeitig mit einem rotatorischen Antrieb 7 erfolgen, der im rohrförmigen Träger 2 angeordnet ist. Günstigerweise kann dazu ein Antrieb 7 mit einer Ringform eingesetzt werden.

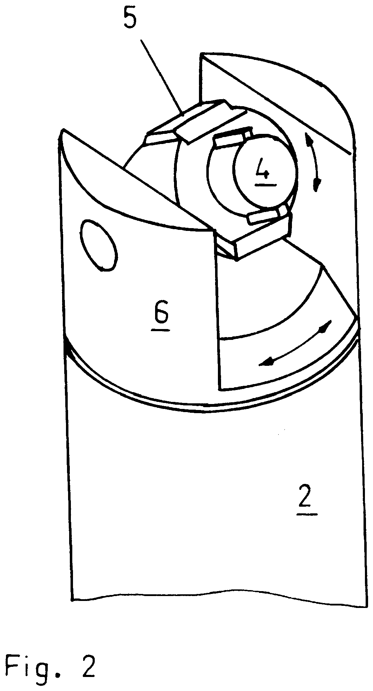

Die Fig. 2 zeigt dazu eine drehbar gelagerte Gabel 6 mit einem drehbar gelagerten Halter 5 mit einer Digitalkamera 4 in einer prinzipiellen Darstellung.

Im rohrförmigen Träger 2 befindet sich der Übertragungsmechanismus zum Schwenken des Halters 5 mit der Digitalkamera 4. Dazu sind der Halter 5 und eine antreibbare Rolle 8 über ein Zugmittelgetriebe miteinander verbunden, wobei die Rolle 8 handbetätigbar ist oder an einen Antrieb gekoppelt ist. Als Zugmittel 9 wird vorzugsweise ein Seil verwendet. Ein Teil des Halters 5 ist dazu eine Rolle für das Zugmittel 9.

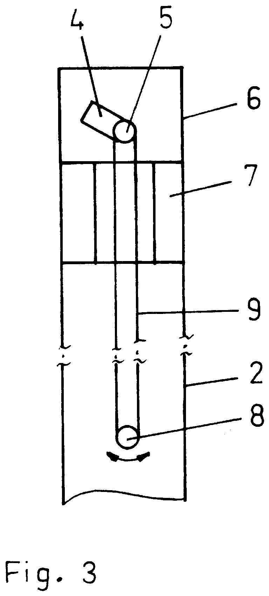

Die Fig. 3 zeigt Antriebe und ein Zugmittelgetriebe für die Gabel 6 und den Halter 5 in einer prinzipiellen Darstellung.
Die Antriebe sind beispielsweise bekannte Schrittmotore, wobei deren Drehwinkel größer 360° ist. Dadurch sind die Drehwinkel der Schrittmotore und damit die Positionen der Gabel 6 und des Halters 5 sowie daraus die Position der Bildfläche der Digitalkamera 4 mittels einer mit den Schrittmotoren verbundenen Steuereinrichtung bestimmbar. Die Bewegung der Digitalkamera 4 erfolgt dazu beispielsweise über handbetätigbare Schalter an dem Griffstück 1 als Bestandteil des rohrförmigen Trägers 2 oder am rohrförmigem Träger 2.

An der Gabel 6 und/oder dem Halter 5 befindet sich wenigstens ein Leuchtmittel für sichtbares Licht.

Das Anschlusskabel für die Digitalkamera 4 kann als flexible Leiterplatte mit Leiterbahnen ausgeführt sein. Damit ist eine Beweglichkeit bei den Bewegungen der Digitalkamera 4 und der Gabel 6 gegeben. Das kann auch durch eine wenigstens bereichsweise spiral- und/oder schraubenförmige Anordnung des Anschlusskabels gewährleistet werden. Die flexible Leiterplatte kann weiterhin Leiterbahnen für die Energieversorgung des wenigstens einen Leuchtmittels aufweisen.

Weiterhin ist eine Haube 3 aus einem für sichtbares Licht transparentem Material für die drehbar gelagerte Gabel 6 einschließlich des Halters 5 mit der Digitalkamera 4 angeordnet. Die Haube 3 ist dazu eine Kugelkappe 3, wobei die Kugelkappe 3 und der rohrförmige Träger 2 dicht miteinander verbunden sind.

In einer Ausführungsform befindet sich der Übertragungsmechanismus in einem rohrförmigen Trägerelement 10 der Gabel 6 zum Schwenken des Halters 5 mit der Digitalkamera 4.

Der besteht dazu aus dem ersten rotatorischen und ringförmigen Antrieb 7 für das rohrförmige Trägerelement 10 und damit der Gabel 6 und einem zweiten Antrieb 11 für den Halter 5.

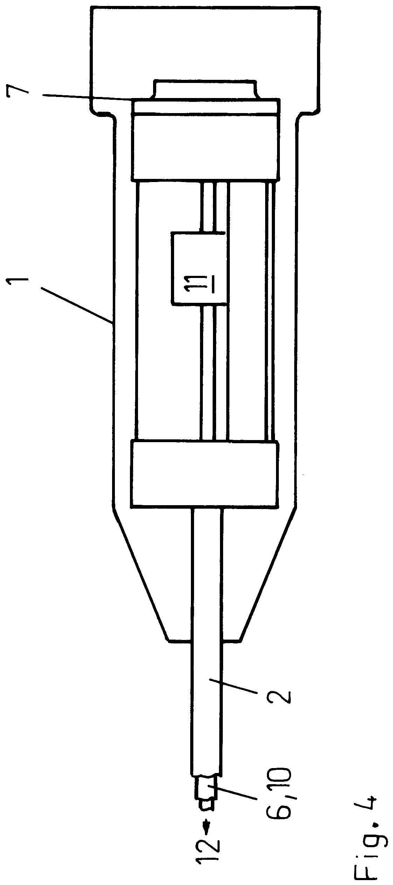

Die Fig. 4 zeigt dazu das Griffstück 1 mit den Antrieben 7, 11 in einer prinzipiellen Darstellung.

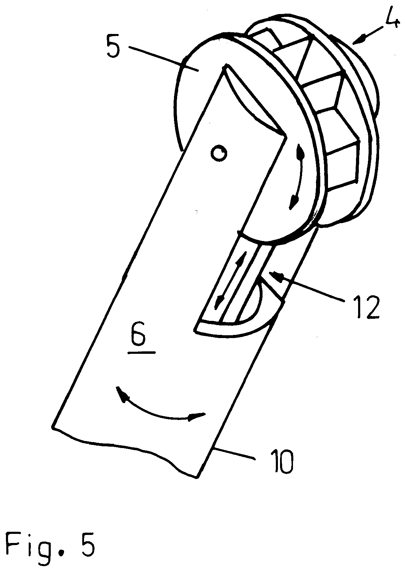

Weiterhin zeigen jeweils in prinzipiellen Darstellungen
die Fig. 5 eine drehbar gelagerte Gabel 6 mit einem drehbar gelagerten Halter mit einer Digitalkamera und

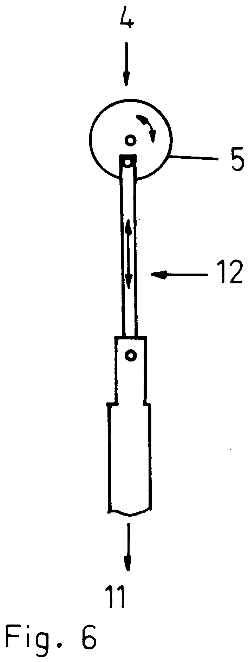

die Fig. 6 einen drehbar gelagerten Halter 5 als Schubgelenk.

Der Übertragungsmechanismus ist eine Schubkurbel 12 wenigstens bereichsweise im rohrförmigen Trägerelement 10. Die Schubkurbel 12 ist an den translatorischen Antrieb 11 als zweiten Antrieb 11 gekoppelt.

Weiterhin sind dieser Antrieb 11 mit der Schubkurbel 12 und das rohrförmige Trägerelement 10 an den ersten rotatorischen und ringförmigen Antrieb 7 gekoppelt. Bei einer Drehbewegung wird damit gleichzeitig der Halter 5 mit der Digitalkamera 4 bewegt.

Das Anschlusskabel für die Digitalkamera 4 befindet sich wenigstens bereichsweise in der Schubkurbel 12 und damit im Inneren des Endoskops. Äquivalent können gleichzeitig die Anschlusskabel für die Leuchtmittel dementsprechend geführt werden.

In einer weiteren Ausführungsform sind die Antriebe mit wenigstens einer fußbetätigbaren Schalteinrichtung verbunden. Das ist beispielsweise eine Wippe mit vier Schaltern für die jeweiligen Bewegungen der Digitalkamera 4 in den durch die beiden Drehachsen vorgegebenen Bewegungsrichtungen. Dabei können die Schalter des Griffstücks 1 entfallen.

In einer weiteren Ausführungsform ist eine Steuereinrichtung in Form eines bekannten Datenverarbeitungssystems mit den Antrieben 7, 11 und einer Vorrichtung zur Blickerfassung verbunden. Die Antriebe werden durch die Bewegung und die Stellung des Auges gesteuert, so dass die durch die Digitalkamera 4 abgebildete Fläche mittels der Augenposition bestimmt ist. Die Kamera zur Bestimmung der Stellung und der Verfolgung der Bewegung wenigstens eines Auges ist dazu ein Bestandteil einer Brille. Die Digitalkamera 4 des Endoskops wird dabei entsprechend der Stellung und der Bewegung des Auges positioniert und bewegt. Dabei können die Schalter des Griffstücks 1 entfallen.

In einer weiteren Ausführungsform ist eine Steuereinrichtung in Form eines bekannten Datenverarbeitungssystems mit den Antrieben 7, 11 und einer Einrichtung zur Gestenerkennung verbunden, so dass die Antriebe 7, 11 durch die Bewegung eines Körperteiles einer Person gesteuert wird.

Die Gestenerkennung basiert auf Sensoren, die von der Person getragen werden, oder mindestens einer Kamera, die die Bewegungen der Person aufnimmt. Im ersten Fall kommen bekannte Beschleunigungs- oder Positionssensoren zum Einsatz. Im zweiten Fall werden die Antriebe 7, 11 durch das von der Person aufgenommene Abbild und einer nachfolgenden bekannten Bildanalyse im Datenverarbeitungssystem gesteuert.

In einer weiteren Ausführungsform kann der Halter 5 gleichzeitig elektrisch leitende Elemente wenigstens zum Betrieb der Digitalkamera 4 in Form der elektrischen Stromversorgung aufweisen. Das sind beispielsweise elektrisch leitende Schichten auf Oberflächenbereichen des Halters 5. Darüber hinaus können Bereiche davon auch Schleifkontakte sein, so dass eine Kontaktierung an der Gabel 6 möglich ist.

## Patentansprüche

1. Endoskop mit
- einem in einer drehbar gelagerten Gabel (6) drehbar gelagerten Halter (5) mit einer Digitalkamera (4), wobei die Gabel (6) radial drehbar so angeordnet ist, dass die Drehachsen der Gabel (6) und des Halters (5) senkrecht zueinander angeordnet sind,
- einer Schubkurbel (12) wenigstens bereichsweise in einem rohrförmigen Trägerelement (10) der Gabel (6) zum Schwenken des Halters (5) mit der Digitalkamera (4),
- einem ersten rotatorischen und ringförmigen Schrittmotor (7), wobei sowohl der Stator als auch der Rotor des Schrittmotors (7) in Form eines Elektromotors ringförmig ausgebildet sind, für das rohrförmige Trägerelement (10) und damit der Gabel (6) und
- einem zweiten an die Schubkurbel (12) gekoppelten translatorischen Schrittmotor (11) für den Halter (5), wobei der translatorische Schrittmotor (11) mit der Schubkurbel und das rohrförmige Trägerelement (10) an den Rotor des ersten rotatorischen und ringförmigen Schrittmotor (7) gekoppelt sind, sich das Anschlusskabel für die Digitalkamera (4) wenigstens bereichsweise in der Schubkurbel (12) und damit im Inneren des Endoskops befindet und die Schrittmotore (7, 11) mit einer Steuereinrichtung verbunden sind, so dass die Positionen der Gabel (6) und des Halters (5) sowie daraus die Position der Bildfläche der Digitalkamera (4) bestimmbar sind, und
- einer Haube (3) aus einem für sichtbares Licht transparentem Material für die drehbar gelagerte Gabel (6) einschließlich dem Halter (5) mit der Digitalkamera (4), wobei die Abmessungen des Endoskopschafts nicht verändert sind.

2. Endoskop nach Patentanspruch 1, **dadurch gekennzeichnet, dass** sich an der Gabel (6) und/oder dem Halter (5) wenigstens ein Leuchtmittel für sichtbares Licht befindet.

3. Endoskop nach Patentanspruch 1, **dadurch gekennzeichnet, dass** das Anschlusskabel eine flexible Leiterplatte ist und dass die flexible Leiterplatte einen flexiblen Träger und Leiterbahnen aufweist.

4. Endoskop nach Patentanspruch 1, **dadurch gekennzeichnet, dass** die Schrittmotore (7, 11) mit wenigstens einer fußbetätigbaren Schalteinrichtung verbunden sind.

5. Endoskop nach Patentanspruch 1, **dadurch gekennzeichnet, dass** eine Steuereinrichtung mit den Schrittmotoren (7, 11) und einer Vorrichtung zur Blickerfassung verbunden ist, so dass die Schrittmore (7, 11) durch die Bewegung des Auges gesteuert wird und die durch die Digitalkamera (4) abgebildete Fläche mittels der Augenposition bestimmt ist.

6. Endoskop nach Patentanspruch 1, **dadurch gekennzeichnet, dass** eine Steuereinrichtung mit den Schrittmotoren (7, 11) und einer Einrichtung zur Gestenerkennung verbunden ist, so dass die Antriebe durch die Bewegung eines Körperteiles gesteuert wird.

7. Endoskop nach Patentanspruch 1, **dadurch gekennzeichnet, dass** der Halter (5) gleichzeitig elektrisch leitende Elemente wenigstens zum Betrieb der Digitalkamera (4) aufweist.

8. Endoskop nach Patentanspruch 1, **dadurch gekennzeichnet, dass** die Haube (3) eine Kugelkappe (3) ist und dass die Kugelkappe (3) und ein Träger dicht miteinander verbunden sind.

9. Endoskop nach Patentanspruch 8, **dadurch gekennzeichnet, dass** wenigstens ein Bereich des Trägers (2) ein starrer oder flexibler Bereich ist.

## Claims

1. Endoscope comprising
- a mount (5), rotatably mounted in a rotatably mounted bracket (6), comprising a digital camera (4), wherein the bracket (6) is radially rotatably arranged such that the axes of rotation of the bracket (6) and the mount (5) are mutually perpendicular,
- a thrust crank (12) at least in regions of a tubular support element (10) of the bracket (6) for pivoting the mount (5) comprising the digital camera (4),
- a first rotary and annular stepper motor (7), wherein both the stator and the rotor of the stepper motor (7) are annular in the form of an electric motor, for the tubular support element (10) and thus the bracket (6) and
- a second translational stepper motor (11), coupled to the thrust crank (12), for the mount (5), wherein the translational stepper motor (11) is coupled to the thrust crank and the tubular support element (10) is coupled to the rotor of the first rotary and annular stepper motor (7), the connecting cable for the digital camera (4) is located, at least in regions, in the thrust crank (12) and thus inside the endoscope, and the stepper motors (7, 11) are connected to a control means such that the positions of the bracket (6) and of the mount (5) can be determined and the position of the image field of the digital camera (4) can be determined therefrom, and
- a cover (3), made of a material that is transparent to visible light, for the rotatably mounted bracket (6) including the mount (5) comprising the digital camera (4), wherein the dimensions of the endoscope shaft are not changed.

2. Endoscope according to claim 1, **characterized in that** at least one lamp for visible light is located on the bracket (6) and/or on the mount (5).

3. Endoscope according to claim 1, **characterized in that** the connecting cable is a flexible printed circuit board and **in that** the flexible printed circuit board comprises a flexible base and conductor tracks.

4. Endoscope according to claim 1, **characterized in that** the stepper motors (7, 11) are connected to at least one foot-operated switching means.

5. Endoscope according to claim 1, **characterized in that** a control means is connected to the stepper motors (7, 11) and to a gaze-tracking device such that the stepper motors (7, 11) are controlled by eye movement and the field displayed by the digital camera (4) is determined by the position of the eye.

6. Endoscope according to claim 1, **characterized in that** a control means is connected to the stepper motors (7, 11) and to a means for gesture recognition such that the drives are controlled by movement of a body part.

7. Endoscope according to claim 1, **characterized in that** the mount (5) comprises simultaneously electrically conductive elements at least for operating the digital camera (4).

8. Endoscope according to claim 1, **characterized in that** the cover (3) is a spherical cap (3) and **in that** the spherical cap (3) and a support are closely interconnected.

9. Endoscope according to claim 8, **characterized in that** at least one region of the support (2) is a rigid or flexible region.

## Revendications

1. Endoscope comprenant
- un support (5) monté de manière rotative dans une fourche (6) montée à rotation et comprenant une caméra numérique (4), la fourche (6) étant disposée de manière à tourner radialement de telle sorte que les axes de rotation de la fourche (6) et du support (5) soient disposés perpendiculairement entre eux,
- une manivelle de poussée (12) placée au moins partiellement dans un élément porteur tubulaire (10) de la fourche (6) pour faire pivoter le support (5) conjointement avec la caméra numérique (4),
- un premier moteur pas à pas rotatif et annulaire (7), le stator et le rotor du moteur pas à pas (7) étant tous les deux formés de manière annulaire sous la forme d'un moteur électrique et étant destinés à l'élément de support tubulaire (10) et la fourche (6) et
- un second moteur pas à pas translatif (11) accouplé à la manivelle de poussée (12) et destiné au support (5), le moteur pas à pas translatif (11) étant accouplé à la manivelle de poussée et l'élément de support tubulaire (10) étant accouplé au rotor du premier moteur pas à pas rotatif et annulaire (7), le câble de raccordement destiné à la caméra numérique (4) se trouvant au moins partiellement dans la manivelle de poussée (12) et donc à l'intérieur de l'endoscope et les moteurs pas à pas (7, 11) étant reliés à un moyen de commande de manière à pouvoir déterminer les positions de la fourche (6) et du support (5) et, en conséquence, la position de la zone d'image de la caméra numérique (4), et
- un capot (3) formé à partir d'un matériau transparent à la lumière visible et destiné à la fourche (6) montée à rotation, y compris le support (5) muni de la caméra numérique (4), les dimensions de l'arbre d'endoscope n'étant pas modifiées.

2. Endoscope selon la revendication 1, **caractérisé en ce qu'**au moins un moyen d'éclairage à lumière visible se trouve au niveau de la fourche (6) et/ou du support (5).

3. Endoscope selon la revendication 1, **caractérisé en ce que** le câble de raccordement est une carte de circuit imprimé flexible et **en ce que** la carte de circuit imprimé flexible comporte un support flexible et des pistes conductrices.

4. Endoscope selon la revendication 1, **caractérisé en ce que** les moteurs pas à pas (7, 11) sont raccordés à au moins un moyen de commutation actionnable par le pied.

5. Endoscope selon la revendication 1, **caractérisé en ce qu'**un moyen de commande est raccordé aux moteurs pas à pas (7, 11) et à un dispositif de détection d'observation de sorte que les moteurs pas à pas (7, 11) sont commandés par le mouvement de l'oeil et la zone formée par la caméra numérique (4) est déterminée au moyen de la position de l'oeil.

6. Endoscope selon la revendication 1, **caractérisé en ce qu'**un moyen de commande est raccordé aux moteurs pas à pas (7, 11) et à un moyen de reconnaissance de gestes de sorte que les entraînements soient commandés par le mouvement d'une partie du corps.

7. Endoscope selon la revendication 1, **caractérisé en ce que** le support (5) comporte en même temps des éléments électriquement conducteurs destinés au moins au fonctionnement de la caméra numérique (4).

8. Endoscope selon la revendication 1, **caractérisé en ce que** le capot (3) est une calotte sphérique (3) et **en ce que** la calotte sphérique (3) et un support sont raccordés l'un à l'autre de manière étanche.

9. Endoscope selon la revendication 8, **caractérisé en ce qu'**au moins une zone du support (2) est une zone rigide ou flexible.
